# EUROPEAN PATENT SPECIFICATION

(11) **EP 4 110 756 B1**
(45) Date of publication and mention of the grant of the patent: **13.08.2025**
(21) Application number: 21759808.5
(22) Date of filing: 19.02.2021
(51) Int. Cl.: C07C 273/18, C07B 63/00, C07C 275/16

(54) **PROCESS FOR THE PURIFICATION OF CARGLUMIC ACID AND INTERMEDIATE OF THIS PROCESS**
VERFAHREN ZUR REINIGUNG VON CARGLUMINSÄURE UND ZWISCHENPRODUKT DIESES VERFAHRENS
PROCÉDÉ DE PURIFICATION D'ACIDE CARGLUMIQUE ET INTERMÉDIAIRE DE CE PROCÉDÉ

(30) Priority: 26.02.2020 CZ 20200100
(43) Date of publication of application: 04.01.2023
(73) Proprietor: SCALE UP LABORATORY, S.R.O., 779 00 Olomouc (CZ)
(72) Inventor: HORÁK, Radim, 789 01 Zábreh (CZ); GREPL, Martin, 783 14 Hlusovice (CZ); HRADIL, Pavel, 783 14 Hlusovice (CZ); FUNK, Petr, 535 01 Prelouc (CZ); KORISTEK, Kamil, 779 00 Samotisky (CZ); KVAPIL, Lubomír, 783 42 (CZ); URBÁSEK, Miroslav, 783 65 (CZ)
(74) Representative: Soukup, Petr
(86) International application number: PCT/CZ2021/000007
(87) International publication number: WO 2021/170154

(56) References cited:
- CN-A- 105 601 542
- CN-A- 107 033 035
- CN-A- 108 017 561
- CN-A- 108 484 448

## Description

### Field of the Invetion

The presented invention belongs into the field of organic chemistry and it describes a method of purification of carglumic acid and an intermediate of this method.

### Prior Art

There is already a wide variety of patents and publications on this topic. Most of them however focus on biologic activity. Carglumic acid and its biologic activity was first described in 1952 in the publication of Grisolia S. et al.: J. Biological Chem. 1952, 198, 561-71. Preparation of its deuterated analog was later described in the Grisolia S. et al,: J. Biological Chem. 1954, 210, 109-17, where a methodology for a similar substance was used, which had been previously described in article Nyc J. et al.: J. Am. Chem. Soc. 1947, 69, 1382. None of these works however dealt with purification of the raw product and its detailed analysis.

The synthesis is described in the newer document CN108484448 A. Reaction of cyanate with glutamic acid in water with pH 11-13 is used according to the scheme 1.

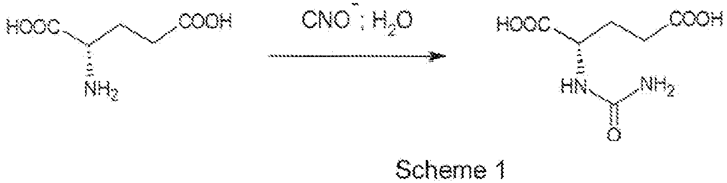

The purity of the final product is 92%. The product is purified through crystallizations and precipitation as in the previous case. The purification through repeated precipitation and crystallizations is also described in the patent CN 108017561 A Repeated precipitation of the product by sulfuric acid and isolation of a highly pure product 99% is also described in the document CN 107033035 A. A very similar procedure, with a more accurate temperature profile, is also described in the patent CN 103980163 A. It however does not deal with purification of the product. Preparation of the compound was described many decades ago. In the current times, essentially the same methods are used, the descriptions are only more detailed. The older works did not deal with the purity of the substance at all, while the newer works described only repeated precipitation, use of various acids and influences of pH, which was described in the patents CN 107033035 A or CN 105601542 A. The maximum purity achieved through these procedures is 99%. Such products are however unsellable, because the acceptable impurity level can reach maximally 0.1% weight percentage. The required higher purity is reached with repetition of the mentioned procedure. This is a time and money consuming process, and moreover, it causes the amount of waste to increase, while the yield decreases.

The aim of the presented invention is to introduce a procedure of purification of carglumic acid - formula I - to high purity through a simple and cheap procedure.

### Summary of the invention

The determined goal is achieved through the invention of a method for obtaining carglumic acid, compound with formula I, 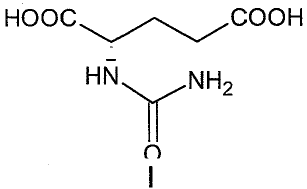 when the raw carglumic acid is dissolved in dimethyl sulfoxide and solvate of carglumic acid and dimethyl sulfoxide is formed. A solvent, in which the solvate of carglumic acid with dimethyl sulfoxide is insoluble, is then added to the generated solution, thereby precipitating the solvate of carglumic acid with dimethyl sulfoxide. The precipitated solvate of carglumic acid with dimethyl sulfoxide is isolated and carglumic acid is subsequently released from it by treatment with water or a mixture of water and an organic or mineral acid, or a mixture of water, an organic acid and a mineral acid. An advantage is pH of reaction mixture in the range from 0 to 5.5. The solvate is characterized with NMR according to the enclosure 1 and 2 and an X-ray powder diffractogram characterized according to the enclosure 3. Raw carglumic acid with formula I was prepared through the reaction of sodium salt of glutamic acid with potassium cyanate according to the scheme 2.

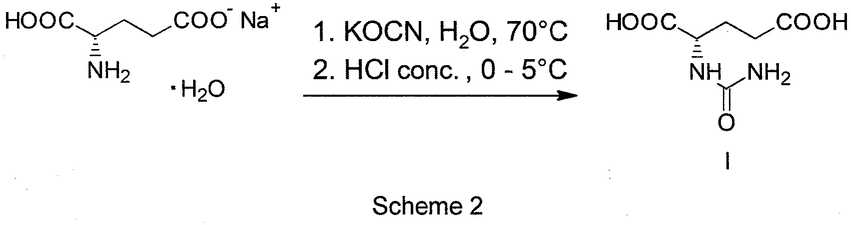

The solvate of carglumic acid with dimethyl sulfoxide is preferably isolated in cold and acetonitrile or a neutral organic solvent with relative permittivity in the range from 2.1 to 29 is used as a precipitating solvent.

The release of carglumic acid from its solvate with dimethyl sulfoxide is preferably carried out with hot water with the addition of a solvent selected from the group of acetone, tetrahydrofuran, methanol, ethanol, 1-propanol, 2-propanol, acetonitrile, formamide, dimethylformamide, dimethylacetamide, N-methylpyrrolidone or mixtures thereof.

Isolated solvate is preferably used for the preparation of pure carglumic acid. Various combinations can be considered. One from the options for solvate decomposition is to use water itself. It is optimal if the desolvation of the solvate takes place at elevated temperatures, ideally when all the crystalline material is dissolved, but the reaction also takes place at temperatures below 10°C, but it takes many times more time. It is possible to use water with organic or mineral acid instead of water. If the decomposition of the solvate does not take place under optimal conditions, carglumic acid of the formula I can be purified through crystallization from water preferably with presence of a solvent. A suitable solvent can be for example: methanol, ethanol, 2-propanol, 1-propanol, acetone, acetonitrile, tetrahydrofuran, dimethylformamide, dimethylacetamide, *N*-methylpyrrolidone, dimethyl sulfoxide.

Another way to release carglumic acid of formula I from its solvate with DMSO is to stir or dissolve the solvate at elevated temperature in an organic acid which can be for example formic acid, acetic acid or propionic acid or in their solution with water or with organic solvent from the group of methanol, ethanol, 2-propanol, 1-propanol, acetone, acetonitrile, tetrahydrofuran, dimethylformamide, dimethylacetamide, N-methylpyrrolidone.

Another option how to release carglumic acid of formula I from its solvate with dimethyl sulfoxide is through stirring or crystallization at elevated temperature in organic solvent from the group - methanol, ethanol, 2-propanol, 1-propanol, acetone, acetonitrile, tetrahydrofuran, dimethylformamide, dimethylacetamide, *N-*methylpyrrolidone, dimethyl sulfoxide with addition of water solution of mineral acid.

Particularly preferable is when the solvate of carglumic acid with dimethyl sulfoxide is dissolved in water at higher temperatures, possibly in a mixture of water with an organic solvent, which can be selected from the group - methanol, ethanol, 2-propanol, 1-propanol, acetone, acetonitrile. The solution can be further purified by filtration with charcoal, kieselguhr or another sorbent. After cooling, pure carglumic acid of the formula I precipitates out of the solution. It is then separated from the reaction mixture, for example by filtration, it is washed with water or a mixture of water with solvents or anhydrous solvents and it is dried.

The subject of the invention is likewise an intermediate of the above mentioned method of purification of carglumic acid which is solvate of carglumic acid with dimethyl sulfoxide NMR characterized according to the enclosure 1 and 2 and x-ray powder diffractogram characterized according to the enclosure 3 with maximal at 36.088; 35.309; 34.866; 31.464; 30.129; 28.865; 27.913; 27.44; 25.838; 24.427; 24.216; 23.654; 22.478; 21.88; 20.607; 19.328; 18.855; 18.562; 17.812; 17.399; 14.929; 11.593; 7.44 ° two-theta.

The presented invention achieves newer and higher efficiency, since the carglumic acid solvate with dimethyl sulfoxide is sparingly soluble in mixtures of dimethyl sulfoxide with above mentioned solvents, but the impurities and concomitant substances which remain in the mother liquors are very soluble in them. The solvate can be further dissolved in water, or mixtures thereof with the above-mentioned solvents and acids, and at this stage it can be further purified by filtration with a suitable absorbent such as charcoal. The use of a carglumic acid solvate with dimethyl sulfoxide is advantageous, mainly due to the simplicity of processing, high yields and cleaning power.
The examples of particular implementations are described below, they do not in any way limit the extent of the protection described in the definition, they solely provide clarification of the disclosure of the invention.

### Enclosure description

Enclosure 1 is ¹H-NMR of the solvate of carglumic acid with dimethyl sulfoxide
Enclosure 2 is ¹³C-NMR of the solvate of carglumic acid with dimethyl sulfoxide
Enclosure 3 is an x ray powder diffractogram of the solvate of carglumic acid with dimethyl sulfoxide

### Examples

### Example 1: Preparation of raw carglumic acid

Into a three necked flask 250 mL equipped with a magnetic stirrer, a thermometer and an air cooler was weighted a monohydrate of monosodium glutamate (37.4 g, 200 mmol), measured water (100 mL) and weighted potassium cyanate (20 g, 246.5 mmol). The suspension was stirred and heated to 70 °C and this temperature was kept for a period of 1 hour. Afterwards, this clear solution was cooled to 0°C and it was dropwise acidified with concentrated hydrochloric acid (app. 38 mL) ensuring the temperature would not exceed 3°C. The suspension was filtrated at this temperature. A filter cake was properly rinsed with ice cold water (3×50 mL), acetone (3×30 mL) at room temperature and dried in vacuum at 35°C. The weight of the raw carglumic acid (white powder) after drying: 28.5 g (75 %), with HPLC purity 95%.

### Example 2: Preparation of solvate of carglumic acid

Into a 250 mL three necked flask equipped with a stirrer, a thermometer, a reflux condenser and dropping funnel was weighted raw product from the example 1 (28.5 g) and added dimethyl sulfoxide (42 mL). The suspension was heated to 90°C until the solid dissolved; the heating was shut down, cooling water was passed through the reflux condenser and acetone (185 mL) was dropped through the dropping funnel into the solution during the period of ten minutes. The formed suspension was cooled to 5 °C, solid material was filtrated off; filter cake was rinsed with acetone (3×30 mL) at room temperature and dried in vacuum in 35 °C. In this way 35.6 g of white crystalline material was received. According to the NMR and HPLC analysis, it is a solvate of carglumic acid with dimethyl sulfoxide. Melting point of the solvate is 112-114 °C.

### Example 3: Preparation of solvate of carglumic acid

The crude product from Example 1 was treated in the same manner as in Example 2, except that Et₂O was added to the solution instead of acetone. A white crystalline solvate of carglumic acid with dimethyl sulfoxide was obtained in a yield of 33.5 g.

### Example 4: Preparation of solvate of carglumic acid

The crude product from Example 1 was treated in the same manner as in Example 2, except that ethyl acetate was added to the solution instead of acetone. A white crystalline solvate of carglumic acid with dimethyl sulfoxide was obtained in a yield of 36.2 g.

### Example 5: Preparation of solvate of carglumic acid

The crude product from Example 1 was treated in the same manner as in Example 2, except that toluene was added to the solution instead of acetone. A white crystalline solvate of carglumic acid with dimethyl sulfoxide was obtained in a yield of 36 g.

### Example 6: Preparation of solvate of carglumic acid

The crude product from Example 1 was treated in the same manner as in Example 2, except that cyclohexanone was added to the solution instead of acetone. A white crystalline solvate of carglumic acid with dimethyl sulfoxide was obtained in a yield of 33 g.

### Example 7: Preparation of solvate of carglumic acid

The crude product from Example 1 was treated in the same manner as in Example 2, except that acetonitrile was added to the solution instead of acetone. A white crystalline solvate of carglumic acid with dimethyl sulfoxide was obtained in a yield of 34 g.

### Example 8: Preparation of solvate of carglumic acid

The crude product from Example 1 was treated in the same manner as in Example 2, except that dichloroethane was added to the solution instead of acetone. A white crystalline solvate of carglumic acid with dimethyl sulfoxide was obtained in a yield of 36 g.

### Eample 9: Desolvation

The crystals of the carglumic acid solvate with dimethyl sulfoxide (35.6 g) were dissolved in a hot mixture of distilled water (106 mL) and acetone (10.8 mL). Charcoal (1.2 g) was added to the solution and after five minutes the solution was filtered through a glass filter. The filtrate was cooled to 40°C and seeded with stirring off. As the needle crystals grew, gentle agitation was started. The temperature of the suspension was allowed to drop spontaneously to 25°C and then it was cooled with water and ice to 0-5°C. After stirring for 1 hour at this temperature, the suspension was filtered and washed with ice-cold distilled water (3×20 mL) and room temperature acetone (3 × 30 mL). The yield of 21.3 g of carglumic acid of formula 1 were received, corresponding to a total yield of 56%. HPLC purity was 99.97%, m.p. 176-178°C.

### Examples 10 - 19: Desolvation

The solvate of carglumic acid with dimethyl sulfoxide from the example 2, was processed in the same manner as in the Example 9 except that a solvent was added to the reaction mixture according to the following table, which shows the total yield of carglumic acid of formula 1 and HPLC purity.

| **Example** | **Solvent** | **Yield (%)** | **HPLC purity (%)** |
|---|---|---|---|
| 10 | Tetrahydrofuran | 50 | 99.94 |
| 11 | Acetonitrile | 55 | 99.91 |
| 12 | Methanol | 45 | 99.95 |
| 13 | Ethanol | 49 | 99.98 |
| 14 | 1-Propanol | 50 | 99.91 |
| 15 | 2-Propanol | 54 | 99.90 |
| 16 | N,N-Dimethylformamide | 49 | 99.98 |
| 17 | N,N-Dimethylacetamide | 53 | 99.91 |
| 18 | N-Methylpyrrolidone | 53 | 99.92 |
| 19 | Formamide | 40 | 99.92 |

### Example 20: Stirring in water

Crystalline solvate of carglumic acid with dimethyl sulfoxide prepared according to the Example 2 (20 g) was stirred up in demineralized water (60 mL) at a temperature of 20 °C. Quantitative desolvation occurred within 24 hours. The solid was filtered off; the filter cake was rinsed with 10% aqueous acetone and the solid was dried in vacuum. The total yield of carglumic acid formula 1 on initial monosodium glutamate was 59 %, HPLC purity 99.2 %.

### Example 21: Crystallization from water

Crystalline solvate of carglumic acid with dimethyl sulfoxide prepared according to the example 2 (20 g) was dissolved in demineralized water (60 mL) at a temperature of 90°C. Crystalline carglumic acid precipitated by spontaneous cooling to room temperature. The suspension was filtered, the filter cake was washed with 10% aqueous acetone and the solid was dried in vacuum. The total yield of carglumic acid of formula 1 per starting sodium glutamate was 46%, HPLC purity 99.57%.

### Examples 22 - 31: Crystallization from water with addition of solvent

The crystalline carglumic acid solvate with dimethyl sulfoxide prepared according to Example 2 (20 g) was dissolved in demineralized water (60 mL) at 90°C. The solution was filtered at 90°C with activated carbon CX (1g). Crystalline carglumic acid precipitated after spontaneous cooling to room temperature. The solvent listed in the following table was added to the precipitated carglumic acid suspension. It also shows the total yield of carglumic acid of formula 1 and the HPLC purity.

| **Example** | **Solvent** | **Yield (%)** | **HPLC purity (%)** |
|---|---|---|---|
| 22 | Tetrahydrofuran | 50 | 99.93 |
| 23 | Acetonitrile | 55 | 99.95 |
| 24 | Methanol | 45 | 99.94 |
| 25 | Ethanol | 49 | 99.91 |
| 26 | 1-Propanol | 50 | 99.91 |
| 27 | 2-Propanol | 54 | 99.95 |
| 28 | N,N-Dimethylformamide | 49 | 99.55 |
| 29 | N,N-Dimethylacetamide | 53 | 99.88 |
| 30 | N-Methylpyrrolidone | 53 | 99.91 |
| 31 | Formamide | 40 | 99.50 |

### Example 32: Crystallization from water and mineral acid

The crystalline carglumic acid solvate with dimethyl sulfoxide prepared according to Example 2 (20 g) was suspended in demineralized water (60 mL) at 20°C. The pH of the suspension was adjusted to 0 with hydrochloric acid (35%). The suspension was heated to 90°C and the resulting solution was filtered with CX activated carbon (1 g). Crystalline carglumic acid precipitated upon spontaneous cooling to room temperature. The suspension was filtered, the filter cake was washed with 10% aqueous acetone and the solid was dried in vacuum. The total yield of carglumic acid of formula 1 per starting sodium glutamate was 43%, HPLC purity 99.12%.

### Example 33: Crystallization from water and mineral acid

The crystalline carglumic acid solvate with dimethyl sulfoxide prepared according to Example 2 (20 g) was suspended in demineralized water (60 mL) at 20°C. The pH of the suspension was adjusted to 0.5 with hydrochloric acid (35%). The suspension was heated to 90°C and the resulting solution was filtered with charcoal CX (1 g). Upon spontaneous cooling to room temperature crystalline carglumic acid precipitated. The suspension was filtered, the filter cake was washed with 10% aqueous acetone and the solid was dried in vacuum. The total yield of carglumic acid of formula 1 per starting sodium glutamate was 45%, HPLC purity 99.41%.

### Example 34: Crystallization from water and organic acid

The crystalline carglumic acid solvate with dimethyl sulfoxide prepared according to Example 2 (20 g) was dissolved in a mixture of demineralized water (60 mL) and concentrated acetic acid (5 mL) at 90°C. The solution was filtered at 90°C with activated carbon CX (1 g). Upon spontaneous cooling to room temperature crystalline carglumic acid precipitated. The suspension was filtered, the filter cake was washed with 10% aqueous acetone and the solid was dried in vacuum. The overall yield of carglumic acid of formula 1 per starting sodium glutamate was 47%, HPLC purity 99.5%.

### Example 35: Stirring in organic acid

The crystalline carglumic acid solvate with dimethyl sulfoxide prepared according to Example 2 (20 g) was stirred in concentrated acetic acid (100 mL) at 90°C. Quantitative desolvation occurred within 1 hour. The suspension was filtered, the filter cake was washed with acetone and the solid was dried in vacuo. The overall yield of carglumic acid of formula 1 per starting sodium glutamate was 50%, HPLC purity 99.50%.

### Example 36: Crystallization from organic acid

The crystalline dimethyl sulfoxide solvate of carglumic acid prepared according to Example 2 (20 g) was dissolved in concentrated acetic acid (300 mL) at 120°C. The solution was filtered at 120°C with activated carbon CX (1 g), the filtrate was cooled to 60°C and the solution was seeded with a crystal of carglumic acid. Upon spontaneous cooling to room temperature crystalline carglumic acid precipitated. The suspension was filtered, the filter cake was washed with acetone and the solid was dried in vacuum. The total yield of carglumic acid of formula 1 per starting sodium glutamate was 40%, HPLC purity 99.48%.

### Example 37: Stirring up in organic acid and water solution on mineral acid

The crystalline dimethyl sulfoxide solvate of carglumic acid prepared according to Example 2 (20 g) was suspended at 20°C in a mixture of concentrated acetic acid (200 mL) and 3% aqueous hydrochloric acid (6 mL). Quantitative desolvation occurred within 1 hour. The suspension was filtered, the filter cake was washed with acetone and the solid was dried in vacuum. The overall yield of carglumic acid of formula 1 per starting sodium glutamate was 55%, HPLC purity 98.93%.

### Example 38: Crystallization from water solution of mineral acid with solvent

The dimethyl sulfoxide solvate of carglumic acid from Example 2 was treated in the same manner as in Example 9, except that 3% aqueous hydrochloric acid was added instead of water to dissolve the solvate. The overall yield of carglumic acid of formula 1 per starting sodium glutamate was 53%, HPLC purity 98.57%.

### Example 39: Stirring up in mixture water, acetone, mineral acid

The crystalline dimethyl sulfoxide solvate of carglumic acid prepared according to Example 2 (20 g) was suspended in a mixture of acetone (60 mL) and 3% aqueous hydrochloric acid (6 mL) at 20° C. Quantitative desolvation occurred within 24 hours. The suspension was filtered, the filter cake was washed with acetone and the solid was dried in vacuum. The overall yield of carglumic acid of formula 1 per starting sodium glutamate was 57%, HPLC purity 98.97%.

### Example 40: Re-purification of polluted carglumic acid

The crystalline dimethyl sulfoxide solvate of carglumic acid prepared according to Example 24 (5 g) was dissolved in water (20 mL) at 90°C, and acetone (2 mL) was added to the resulting solution. The solution was filtered with charcoal at 50°C through a pad of Celite. Free cooling to room temperature precipitated needle-like crystals, which were filtered off and washed with 3 x 2 mL of ice water and 3 x 2 mL of acetone. The obtained product was dried at 40°C. The yield 4 g of carglumic acid of formula 1 were obtained with an HPLC purity of 99.97%.

### Industrial applicability

The invention is useful in the pharmaceutical industry in preparation of important medicaments based on carglumic acid.

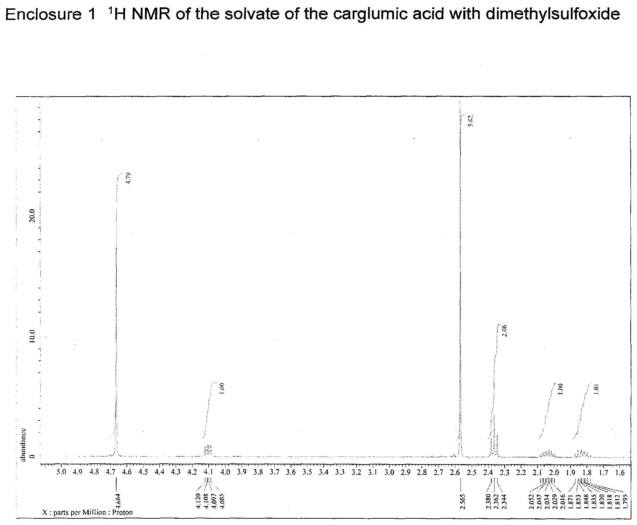

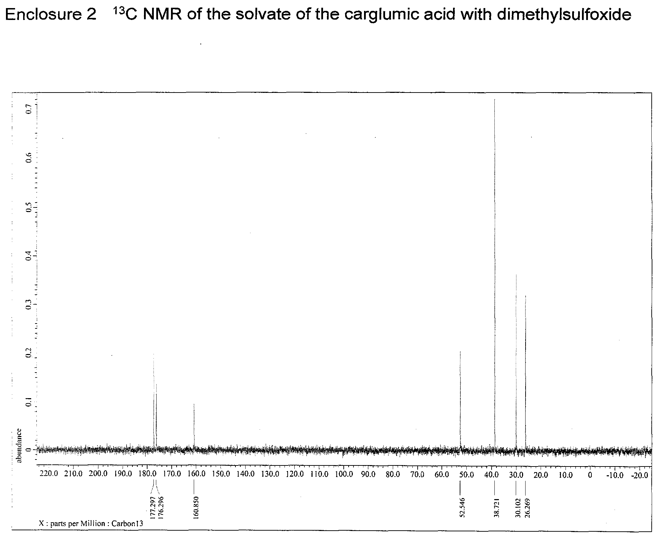

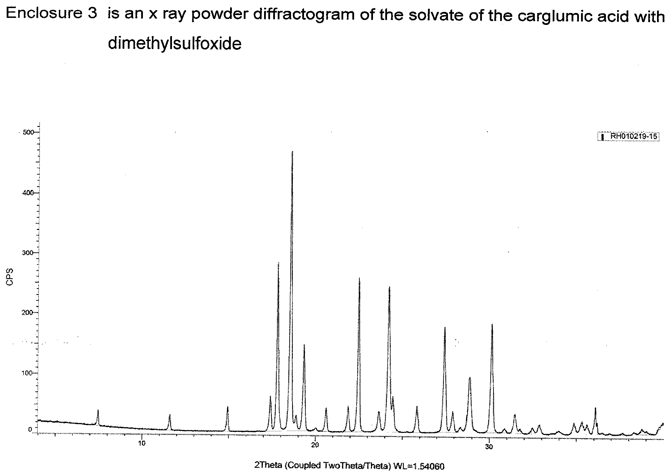

## Claims

1. Method of purification of carglumic acid, substance formula I, 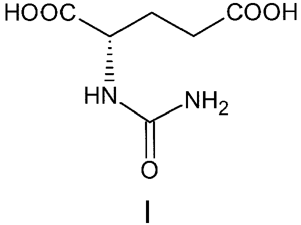 **wherein,** it contains the following steps
a) Dissolution of carglumic acid in dimethyl sulfoxide
b) Addition of a precipitating solvent
c) Isolation of the precipitated carglumic acid solvate with dimethyl sulfoxide,
d) Release of carglumic acid from the solvate through treatment with water, optionally a mixture of water and an organic solvent, optionally a mixture of water and an organic or mineral acid, optionally a mixture of water, organic acid and mineral acid, optionally with an organic acid, optionally with a mixture of water, mineral acid and organic solvent.

2. The method according to the claim 1, **wherein** acetonitrile or a neutral organic solvent with relative permittivity in the range 2,1 - 29 is used as the precipitation solvent.

3. The method according to the claim 2, **wherein** acetone is used as the precipitation solvent.

4. The method according to the claim 1, **wherein** the isolated solvate of carglumic acid with dimethyl sulfoxide through heating in water, eventually in a mixture of water and solvent or solvents which are selected from the group - acetone, tetrahydrofuran, methanol, ethanol, 1-propanol, 2-propanol, acetonitrile, formamide, N,N-dimethylformamide, N,N-dimethylacetamide, N-methylpyrrolidone, is transformed into a solution, charcoal is added, subsequently the active coal is separated away and then the obtained filtrate is cooled and the precipitated carglumic acid formula I is then isolated.

5. The method according to the claim 1, **wherein** into water suspension of carglumic acid after decomposition of its solvate with dimethylsulfoxide is added a solvent which is selected from the group acetone, tetrahydrofuran, methanol, ethanol, 1-propanol, 2-propanol, acetonitrile, formamide, N,N-dimethylformamide, N,N-dimethylacetamide, N-methylpyrrolidone or their mixture.

6. The method according to the claim 5, **wherein** acetone is used as a solvent.

7. The solvate of the carglumic acid with dimethyl sulfoxide which is **characterized by** an x ray powder diffractogram according with peaks at 36.088; 35.309; 34.866; 31.464; 30.129; 28.865; 27.913; 27.44; 25.838; 24.427; 24.216; 23.654; 22.478; 21.88; 20.607; 19.328; 18.855; 18.562; 17.812; 17.399; 14.929; 11.593; 7.44 ° two-theta.

## Patentansprüche

1. Verfahren zur Reinigung von Carglumsäure, Substanzformel I, 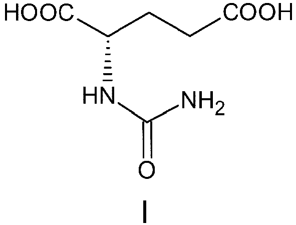 **dadurch gekennzeichnet,** dasses die folgenden Schritte enthält
a) Auflösung von Carglumsäure in Dimethylsulfoxid
b) Zugabe eines Fällungslösungsmittels
c) Isolierung des gefällten Carglumsäure-Solvats mit Dimethylsulfoxid,
d) Freisetzung der Carglumsäure aus dem Solvat durch Behandlung mit Wasser; gegebenenfalls mit einem Gemisch aus Wasser und einem organischen Lösungsmittel; gegebenenfalls mit einem Gemisch aus Wasser und einer organischen oder Mineralsäure; gegebenenfalls mit einem Gemisch aus Wasser, organischer Säure und Mineralsäure; gegebenenfalls mit einer organischen Säure; gegebenenfalls mit einem Gemisch aus Wasser, Mineralsäure und organischem Lösungsmittel.

2. Verfahren nach Anspruch 1, **dadurch gekennzeichnet, dass** Acetonitril oder ein neutrales organisches Lösungsmittel mit einer relativen Dielektrizitätskonstante im Bereich zwischen 2,1 und 29 als Fällungslösungsmittel verwendet wird.

3. Verfahren nach Anspruch 2, **dadurch gekennzeichnet, dass** Aceton als Fällungslösungsmittel verwendet wird.

4. Verfahren nach Anspruch 1, **dadurch gekennzeichnet, dass** das isolierte Solvat der Carglumsäure mit Dimethylsulfoxid durch Erhitzen in Wasser, gegebenenfalls in einem Gemisch aus Wasser und Lösungsmittel oder Lösungsmitteln, die aus der Gruppe Aceton, Tetrahydrofuran, Methanol, Ethanol, 1-Propanol, 2-Propanol, Acetonitril, Formamid, N,N-Dimethylformamid, N,N-Dimethylacetamid, N-Methyl-2-pyrrolidon ausgewählt sind, in eine Lösung überführt und Aktivkohle zugesetzt wird, anschließend die Aktivkohle abgetrennt und das erhaltene Filtrat abgekühlt und die ausgefällte Carglumsäure Formel I isoliert wird.

5. Verfahren nach Anspruch 1, **dadurch gekennzeichnet, dass** zu einer wässrigen Suspension von Carglumsäure nach Zersetzung ihres Solvats mit Dimethylsulfoxid ein Lösungsmittel zugegeben wird, das aus der Gruppe Aceton, Tetrahydrofuran, Methanol, Ethanol, 1-Propanol, 2-Propanol, Acetonitril, Formamid, N,N-Dimethylformamid, N,N-Dimethylacetamid, N-Methyl-2-pyrrolidon oder deren Gemisch ausgewählt ist.

6. Verfahren nach Anspruch 5, **dadurch gekennzeichnet, dass** Aceton als Lösungsmittel verwendet wird.

7. Das Solvat der Carglumsäure mit Dimethylsulfoxid, das durch ein Röntgenpulverdiffraktogramm mit Peaks bei den Braggschen Winkeln 36,088; 35,309; 34,866; 31,464; 30,129; 28,865; 27,913; 27,44; 25,838; 24,427; 24,216; 23,654; 22,478; 21,88; 20,607; 19,328; 18,855; 18,562; 17,812; 17,399; 14,929; 11,593; 7,44 (Zwei-Theta-Werte) gekennzeichnet ist.

## Revendications

1. Méthode de purification de l'acide carglumique, substance de formule I, 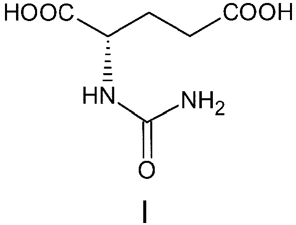 **dans laquelle** on trouve les étapes suivantes :
a) Dissolution de l'acide carglumique dans le sulfoxyde de diméthyle ;
b) Ajout d'un solvant précipitant ;
c) Isolement du solvate d'acide carglumique précipité avec du sulfoxyde de diméthyle ;
d) Libération de l'acide carglumique du solvate par traitement à l'eau, éventuellement par un mélange d'eau et de solvant organique, éventuellement par un mélange d'eau et d'acide organique ou minéral, éventuellement par un mélange d'eau, d'acide organique et d'acide minéral, éventuellement par un acide organique, éventuellement par un mélange d'eau, d'acide minéral et de solvant organique.

2. Méthode selon la revendication 1, **dans laquelle** l'acétonitrile ou un solvant organique neutre dont la permittivité relative est comprise entre 2,1 et 29 est utilisé comme solvant de précipitation.

3. Méthode selon la revendication 2, **dans laquelle** l'acétone est utilisée comme solvant de précipitation.

4. La méthode selon la revendication 1, **dans laquelle** le solvate isolé de l'acide carglumique avec le sulfoxyde de diméthyle par chauffage dans l'eau, éventuellement dans un mélange d'eau et de solvant ou de solvants qui sont choisis dans le groupe acétone, tétrahydrofurane, méthanol, éthanol, propan-1-ol, propan-2-ol, acétonitrile, formamide, N,N-diméthylformamide, N,N-diméthylacétamide, N-méthylpyrrolidone, est transformé en solution, du charbon de bois est ajouté, le charbon actif est ensuite séparé et le filtrat obtenu est refroidi et l'acide carglumique précipité de formule I est alors isolé.

5. Méthode selon la revendication 1, **dans laquelle** un solvant choisi dans le groupe acétone, tétrahydrofurane, méthanol, éthanol, propan-1-ol, propan-2-ol, acétonitrile, formamide, N,N-diméthylformamide, N,N-diméthylacétamide, N-méthylpyrrolidone ou leur mélange est ajouté à la suspension aqueuse de l'acide carglumique après décomposition de son solvant avec le diméthylsulfoxyde.

6. Méthode selon la revendication 5, **dans laquelle** l'acétone est utilisée comme solvant.

7. Le solvate de l'acide carglumique avec le diméthylsulfoxyde qui est **caractérisé par** un diffractogramme de poudre de rayons X selon les pics à 36.088 ; 35.309 ; 34.866 ; 31.464 ; 30.129 ; 28.865 ; 27.913 ; 27.44 ; 25.838 ; 24.427 ; 24.216 ; 23.654 ; 22.478 ; 21.88 ; 20.607 ; 19.328 ; 18.855 ; 18.562 ; 17.812 ; 17.399 ; 14.929 ; 11.593 ; 7.44 ° deux-thêta.
